# EUROPÄISCHE PATENTSCHRIFT

(11) **EP 4 118 567 B1**
(45) Veröffentlichungstag und Bekanntmachung des Hinweises auf die Patenterteilung: **18.09.2024**
(21) Anmeldenummer: 21716301.3
(22) Anmeldetag: 26.03.2021
(51) Int. Cl.: G16H 30/20, G16H 30/40, G16H 40/63, G16H 40/67

(54) **TELENOTARZTSYSTEM UND VERFAHREN ZUM BETRIEB EINES TELENOTARZTSYSTEMS**
REMOTE EMERGENCY DOCTOR SYSTEM AND METHOD FOR OPERATING A REMOTE EMERGENCY DOCTOR SYSTEM
SYSTÈME DE MÉDECIN D'URGENCE À DISTANCE ET PROCÉDÉ DE FONCTIONNEMENT D'UN SYSTÈME DE MÉDECIN D'URGENCE À DISTANCE

(30) Priorität: 01.04.2020 DE 102020109040
(43) Veröffentlichungstag der Anmeldung: 18.01.2023
(73) Patentinhaber: peiker Holding GmbH, 61352 Bad Homburg (DE)
(72) Erfinder: OTTO, Thorsten, 12347 Berlin (DE); TIMME, Sebastian, 12169 Berlin (DE)
(74) Vertreter: Otten, Roth, Dobler & Partner mbB Patentanwälte
(86) Internationale Anmeldenummer: PCT/EP2021/057863
(87) Internationale Veröffentlichungsnummer: WO 2021/198048

(56) Entgegenhaltungen:
- JP-B2- 4 676 146
- US-A1- 2008 146 277
- SEBASTIAN BERGRATH ET AL: "Implementation phase of a multicentre prehospital telemedicine system to support paramedics: feasibility and possible limitations", SCANDINAVIAN JOURNAL OF TRAUMA, RESUSCITATION AND EMERGENCY MEDICINE, BIOMED CENTRAL LTD, LONDON UK, vol. 21, no. 1, 11 July 2013 (2013-07-11), pages 54, XP021155886, ISSN: 1757-7241, DOI: 10.1186/1757-7241-21-54
- BROKMANN J.C. ET AL: "Telemedicine : Perspektiven für die ländliche Notfallversorgung : Perspectives for emergency medical care in rural areas", NOTFALL + RETTUNGSMEDIZIN, vol. 17, no. 3, 10 April 2014 (2014-04-10), Berlin/Heidelberg, pages 209 - 216, XP055812927, ISSN: 1434-6222, DOI: 10.1007/s10049-013-1791-x

## Beschreibung

Die Erfindung betrifft ein Telenotarztsystem gemäß dem Oberbegriff des Anspruchs 1 sowie ein Verfahren zum Betrieb eines Telenotarztsystems gemäß Anspruch 5.

Systeme, die in einem Rettungswagen installierte Kameras und am Körper von Rettungssanitätern getragene Kameras kombinieren, um mittels Live-Video vom Einsatzort die Rettungssanitäter beim Einsatz durch einen nicht am Einsatzort befindlichen Notarzt zu unterstützen, sind gegenwärtig in der Erprobung. Ein derartiges System wird zum Beispiel unter https://www.iqmx.eu/innovat.ionen/telenotarzt/ als "Referenzprojekt Telenotarzt Bayern" der IQ Medworks GmbH beschrieben.

Aus der US 2008/146277 A1 sind Verfahren und Vorrichtungen zum Bereitstellen von Gesundheitsfürsorge aus der Ferne offenbart. Eine Ausführungsform umfasst einen Transceiver, der eine Kamera, eine Anzeige, einen Lautsprecher, ein Mikrofon und eine eingebettete Fernbedienung umfasst. Dieser Transceiver kann zu Hause, bei der Arbeit, auf Reisen oder an jedem anderen Ort verwendet werden, der drahtgebundenen oder drahtlosen Zugang zu einem Netzwerk bietet, wie z. B. dem Internet oder einem Mobiltelefonsystem. Der Transceiver kann verwendet werden, um Informationen, Behandlung oder medizinische Versorgung von einem Gesundheitsdienstleister zu erhalten. In einer Ausführungsform umfasst der Transceiver Diagnose- und Behandlungssoftware. In einer anderen alternativen Ausführungsform kann auch eine Vielzahl von Datengeräten umfasst sein, die über eine drahtgebundene oder drahtlose Verbindung mit dem Mobiltelefon verbunden sind. In einer Ausführungsform kann ein Gesundheitsdienstleister oder eine Gesundheitseinrichtung den Transceiver und/oder ein Datengerät teilweise oder gemeinsam steuern.

Unter der Dokumentennummer XP021155886 ist ein Fachbericht verfügbar, welcher eine Implementierungsphase eines multizentrischen prähospitalen Telemedizinsystems zur Unterstützung von Rettungssanitätern beschreibt.

Unter der Dokumentennummer XP055812927 ist ein Fachbericht verfügbar, welcher Perspektiven für die ländliche Notfallversorgung beschreibt.

Aus der JP 4 676146 B2 ist es bekannt eine Vielzahl von Videodaten zusammen mit Audiodaten zu speichern und zu verteilen, sowie ein Telekonferenzsystem oder dergleichen zu konstruieren.

Es ist Aufgabe der Erfindung, ein Telenotarztsystem vorzuschlagen, welches eine unterbrechungsfreie telemedizinische Versorgung eines Patienten bei gleichzeitig jeweils bestmöglicher Bildqualität zulässt. Weiterhin ist es Aufgabe der Erfindung ein Verfahren zum Betrieb eines Telenotarztsystems vorzuschlagen, welches eine unterbrechungsfreie telemedizinische Versorgung eines Patienten bei gleichzeitig jeweils bestmöglicher Bildqualität ermöglicht.

Diese Aufgabe wird ausgehend von den Merkmalen des Oberbegriffs des Anspruchs 1 bzw. 5 durch die kennzeichnenden Merkmale des Anspruchs 1 bzw. 5 gelöst. In den jeweiligen Unteransprüchen sind vorteilhafte und zweckmäßige Weiterbildungen angegeben.

Das erfindungsgemäße Telenotarztsystem umfasst
- wenigstens eine in einem Fahrzeug angeordnete Digitalkamera,
- wenigstens eine tragbare Digitalkamera, die von einem Nutzer getragen ist,
- ein Überwachungsmodul, welches einen Bildschirm und ein Fernsteuereinheit umfasst,
- wenigstens einen Router, mit welchem die Digitalkameras verbunden sind,
- einen Server, mit welchem der wenigstens eine Router und das Überwachungsmodul verbunden sind,
- wobei die wenigstens eine in dem Fahrzeug angeordnete Digitalkamera als Steuermechanismen einen Zoom-Mechanismus und/oder einen Schwenk- /Drehmechanismus umfasst,
- wobei die Steuermechanismen der wenigstens einen in dem Fahrzeug angeordneten Digitalkamera mittels der Fernsteuereinheit des Überwachungsmoduls ferngesteuert sind,
- wobei die wenigstens eine tragbare Digitalkamera oder wenigstens einer der Router oder der Sever auf der Basis der von der wenigstens einen tragbaren Digitalkamera aufgenommenen Digitalbilder eine Kollektion wesentlicher Merkmale wenigstens eines Patienten generiert, wobei die Kollektion wesentlicher Merkmale insbesondere aus einem Gesicht des Patienten generiert ist,
- wobei ein Algorithmus die Kollektion der wesentlichen Merkmale des Patienten mit den laufend in dem Fahrzeug von der wenigstens einen Digitalkamera aufgenommenen Digitalbildern derart vergleicht, dass das Telenotarztsystem eine Übertragung der in dem Fahrzeug aufgenommenen Digitalbilder an den Bildschirm des Überwachungsmoduls startet, sobald ein Vergleich ergibt, dass einer der von der tragbaren Digitalkamera erfassten Patienten von der in dem Fahrzeug angeordneten Digitalkamera erfasst ist,
- wobei der Algorithmus in wenigstens eine der Digitalkameras des Fahrzeugs oder in den wenigstens einen der Router oder in den Server implementiert ist.

Mit einem derartigen Telenotarztsystem ist es möglich, eine unterbrechungsfreie telemedizinische Versorgung eines Patienten bei gleichzeitig jeweils bestmöglicher Bildqualität zur Verfügung zu stellen, da eine automatische Umschaltung von der tragbaren Digitalkamera auf die in dem Fahrzeug verbaute Digitalkamera erfolgt, sobald der Patient von der im Fahrzeug verbauten Digitalkamera erkannt ist. Somit werden die sich in der Regel stark bewegenden, von der tragbaren Digitalkamera, welche als Bodycam ausgebildet ist, aufgenommenen Digitalbilder so schnell wie möglich durch weniger bewegte Digitalbilder der in dem Fahrzeug verbauten Digitalkamera ersetzt. Hierbei erfolgt eine derartige Umschaltung von den Digitalbildern der tragbaren Digitalkamera auf die Digitalbilder der fest in dem Fahrzeug verbauten Digitalkamera automatisiert auf der Basis einer Erkennung des Patienten. Somit ist sichergestellt, dass die Umschaltung weder vergessen oder übersehen werden kann, noch dass die Umschaltung einen Arbeitsaufwand erfordert.

Weiterhin kann es vorgesehen sein, das Telenotarztsystem mit einer medizintechnischen Ausrüstung auszustatten, wobei diese außerhalb des Fahrzeugs angeordnet ist, wobei diese zur Erfassung von Vitaldaten des Patienten geeignet ist und wobei diese über den wenigstens einen Router mit dem Server derart verbunden ist, dass die erfassten Vitaldaten an den Server übertragen werden. Somit kann bereits außerhalb des Fahrzeugs eine medizinische Versorgung vorgenommen werden, bei welcher der Telenotarzt, zusätzlich zu den von der tragbaren Digitalkamera aufgenommenen Digitalbildern, über Vitaldaten des Patienten verfügt.

Weiterhin kann vorgesehen sein, dass der wenigstens eine Router mit dem Server drahtlos kommuniziert. Hierdurch ist das Telenotarztsystem unabhängig von einer festen Verkabelung.

Es kann auch vorgesehen sein, dass das Telenotarztsystem zwei Router umfasst, wobei die in dem Fahrzeug angeordnete wenigstens eine Digitalkamera mit dem ersten Router verbunden ist, wobei der erste Router in dem Fahrzeug angeordnet ist, wobei wenigstens eine der von den Nutzern getragenen Digitalkameras mit dem zweiten Router verbunden ist, wobei der zweite Router als portabler Router ausgebildet ist und im Einsatz außerhalb des Fahrzeugs angeordnet ist. Hierdurch werden die Übertragungswege zwischen den Digitalkameras und den Routern kurz gehalten und hierdurch Störungen bei der Übertagung zuverlässiger vermieden. Weiterhin ist ein derartiges System auch für die Versorgung eines Patienten in einem schwer zugänglichen Gelände geeignet, in welchem der Patient zunächst weit entfernt vom Fahrzeug versorgt werden muss.

Das erfindungsgemäße Verfahren zum Betrieb eines Telenotarztsystems, welches nach wenigstens einem der Ansprüche 1 bis 4 ausgebildet ist, umfasst die Schritte:
- Filmen eines Patienten mit einer von einem Nutzer getragenen tragbaren Digitalkamera;
- Generieren einer Kollektion wesentlicher Merkmale eines Patienten auf der Basis der durch das Filmen erfassten Digitalbilder, wobei die Kollektion wesentlicher Merkmale insbesondere aus einem Gesicht des Patienten generiert wird und wobei die Generierung der Kollektion durch die tragbare Digitalkamera oder durch wenigstens einen der Router oder durch den Sever erfolgt;
- Vergleichen der Kollektion der wesentlichen Merkmale des Patienten mit den laufend in dem Fahrzeug von einer dortigen Digitalkamera aufgenommenen Digitalbildern mittels eines Algorithmus, wobei der Algorithmus in die Digitalkameras des Fahrzeugs oder in den Router oder in den Server implementiert ist;
- Starten einer Übertragung der in dem Fahrzeug aufgenommenen Digitalbilder an einen Bildschirm eines Überwachungsmoduls sobald das Vergleichen zu dem Ergebnis führt, dass der von der tragbaren Digitalkamera bereits erfasste Patient von der in dem Fahrzeug angeordneten Digitalkamera erfasst wird.

Mit einem derartigen Verfahren ist es möglich, eine unterbrechungsfreie telemedizinische Versorgung eines Patienten bei gleichzeitig jeweils bestmöglicher Bildqualität zur Verfügung zu stellen, da eine automatische Umschaltung von der tragbaren Digitalkamera auf die in dem Fahrzeug verbaute Digitalkamera erfolgt, sobald der Patient durch diese erkannt wird. Somit werden die sich in der Regel stark bewegenden, von der tragbaren Digitalkamera, welche als Bodycam ausgebildet ist, aufgenommenen Digitalbilder durch weniger bewegte Digitalbilder der in dem Fahrzeug verbauten Digitalkamera ersetzt. Hierbei erfolgt eine derartige Umschaltung von den Digitalbildern der tragbaren Digitalkamera auf die Digitalbilder der fest in dem Fahrzeug verbauten Digitalkamera automatisiert auf der Basis einer Erkennung des Patienten. Somit ist sichergestellt, dass die Umschaltung weder vergessen werden kann, noch dass die Umschaltung einen Abreitsaufwand mit sich bringt.

Weiterhin kann es vorgesehen sein, das erfindungsgemäße Verfahren um die nachfolgend genannten, zusätzlichen Schritte zu ergänzen:
- Erfassen von Vitaldaten des Patienten während der Patient von dem Nutzer mit der tragbaren Digitalkamera aufgenommen wird;
- Übermitteln der Vitaldaten des Patienten zu dem Router und von dem Router zu dem Server. Diese Schritte ermöglichen es, dass bereits außerhalb des Fahrzeugs eine medizinische Versorgung vorgenommen wird, bei welcher der Telenotarzt, zusätzlich zu den von der tragbaren Digitalkamera aufgenommenen Digitalbildern, über Vitaldaten des Patienten verfügt.

Es kann auch vorgesehen sein, das Verfahren noch um einen weiteren Schritt zu ergänzen, nämlich durch das Einblenden der an den Server übermittelten Vitaldaten des Patienten in ein auf einem Bildschirm eines Überwachungsmoduls angezeigtes Bild des Patienten. Hierdurch ist eine umfassende Information des Telenotarztes gewährleistet.

Weiterhin kann es vorgesehen sein, das Verfahren durch den nachfolgend ausgeführten, zusätzlichen Schritt zu ergänzen:
- Aktivieren wenigstens einer der in dem Fahrzeug angeordneten Digitalkameras durch Entnahme einer Trage aus dem Fahrzeug. Durch diesen Schritt wird sichergestellt, dass das Telenotarztsystem im Einsatz rechtzeitig vollständig hochgefahren wird, so dass die im Fahrzeug angeordnete Digitalkamera bzw. die im Fahrzeug angeordneten Digitalkameras einen in das Fahrzeug geschobenen Patienten erfassen und erkennen können.

Alternativ zu dem vorgehend genannten Schritt kann es auch vorgesehen sein, dass eine Aktivierung der wenigstens einen in dem Fahrzeug angeordneten Digitalkamera durch Aktivierung einer Live-Video Funktion an einer der tragbaren Digitalkameras erfolgt. Hierdurch kann ebenfalls sicher gestellt werden, dass die im Fahrzeug angeordnete Digitalkamera bzw. die im Fahrzeug angeordneten Digitalkameras einen in das Fahrzeug geschobenen Patienten erfassen und erkennen können.

Schließlich ist es vorgesehen, zum Betrieb eines Telenotarztsystems, nach wenigstens einem der vorgenannten Verfahrensansprüche:
- Vitaldaten mit einer medizinischen Vorrichtung wie zum Beispiel insbesondere einem Stethoskop zu erfassen,
- die Vitaldaten von der medizinischen Vorrichtung an eine in einem Fahrzeug angeordnete Digitalkamera per Funk, insbesondere per Bluetooth zu übertragen und
- die Vitaldaten über einen Audiokanal der Digitalkamera an einen Router weiterzuleiten.

Ein derartiges Vorgehen ermöglicht eine Einbindung von medizinischen Vorrichtungen mit schwacher Sendeleistung, da sich Digitalkamera und medizinische Vorrichtung im Betrieb immer in der Nähe des Patienten und somit in räumlicher Nähe zueinander befinden. Weiterhin wird durch eine Übertragung der Vitaldaten über den Audiokanal eine Beeinträchtigung der Bilddaten zuverlässig vermieden.

Weitere Einzelheiten der Erfindung werden in der Zeichnung anhand von schematisch dargestellten Ausführungsbeispielen beschrieben. Hierbei zeigt:
- Figur 1:: zeigt eine erste Ausführungsvariante eines erfindungsgemäßen Telenotarztsystems;
- Figur 2:: zeigt eine zweite Ausführungsvariante eines erfindungsgemäßen Telenotarztsystems;
- Figur 3:: zeigt eine dritte Ausführungsvariante eines erfindungsgemäßen Telenotarztsystems und
- Figur 4:: zeigt eine vierte Ausführungsvariante eines erfindungsgemäßen Telenotarztsystems.

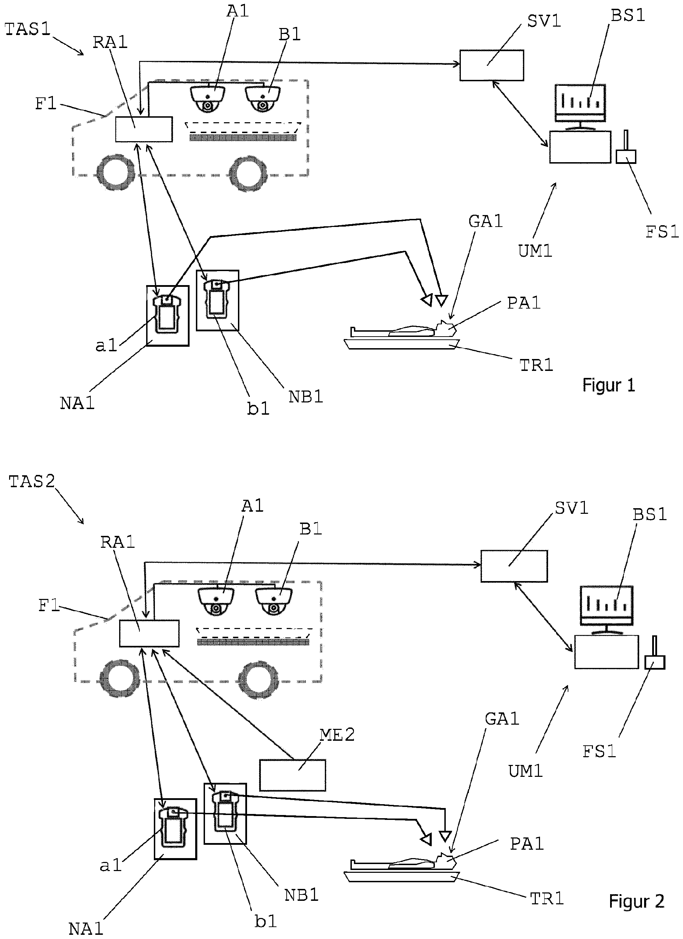

In der Figur 1 ist eine erste Ausführungsvariante eines erfindungsgemäßen Telenotarztsystems TAS1 gezeigt, welches einen Router RA1 umfasst. Das erste Telenotarztsystem TAS1 umfasst eine erste in einem Fahrzeug F1 angeordnete Digitalkamera A1 und eine zweite in dem Fahrzeug F1 angeordnete Kamera B1. Weiterhin umfasst das Telenotarztsystem TAS1 zwei tragbare Digitalkameras a1, b1, wobei ein Nutzer NA1 die erste tragbare Digitalkamera a1 trägt und wobei ein Nutzer NB1 die zweite Digitalkamera b1 trägt. Die Nutzer NA1 und NB1 sind rein schematisch jeweils als Rechteck dargestellt. Die Nutzer sind Rettungssanitäter und tragen die tragbare Digitalkamera üblicher Weise von der Brust oder am Helm, so dass der Patient bei der Versorgung in dem Bereich von dem Sanitäter aufgenommen wird, welcher vor der Brust oder vor dem Kopf des Sanitäters liegt. Das Telenotarztsystem TAS1 umfasst auch ein Überwachungsmodul UM1, welches einen Bildschirm BS1 und eine Fernsteuereinheit FS1 umfasst. Weiterhin umfasst Telenotarztsystem TAS1 den erwähnten Router RA1, mit welchem die im Fahrzeug F1 angeordneten Digitalkameras A1, B1 und die tragbaren Digitalkameras a1, b1 verbunden sind. Schließlich umfasst das Telenotarztsystem TAS1 einen Server SV1, mit welchem der Router RA1 und das Überwachungsmodul UM1 verbunden sind. Beide in dem Fahrzeug F1 angeordneten Digitalkameras A1, B1 umfassen als Steuermechanismen einen Zoom-Mechanismus und einen Schwenk-/Drehmechanismus, wobei die Steuermechanismen der in dem Fahrzeug F1 angeordneten Digitalkameras A1, B1 mittels der Fernsteuereinheit FS1 des Überwachungsmoduls UM1 ferngesteuert sind. Der Sever SV1 generiert auf der Basis der von den tragbaren Digitalkameras a1, b1 aufgenommenen Digitalbilder eine Kollektionen wesentlicher Merkmale eines Patienten PA1, wobei die Kollektion wesentlicher Merkmale insbesondere jeweils aus einem Gesicht GA1 des Patienten PA1 erzeugt ist. Ein Algorithmus vergleicht die Kollektion der wesentlichen Merkmale des Patienten PA1 mit den laufend in dem Fahrzeug F1 von den beiden Digitalkameras A1, B1 aufgenommenen Digitalbildern derart, dass das Telenotarztsystem TAS1 eine Übertragung der in dem Fahrzeug F1 aufgenommenen Digitalbilder an den Bildschirm BS1 des Überwachungsmoduls UM1 startet, wenn ein Vergleich ergibt, dass der von den tragbaren Digitalkameras a1, b1 erfasste Patient PA1 von einer der in dem Fahrzeug F1 angeordneten Digitalkameras A1, B1 erfasst ist. Hierbei ist der Algorithmus in den Router RA1 implementiert. Der Router RA1 ist mit dem Server SV1 drahtlos über ein Mobilfunknetzwerk oder über Satellitenfunk verbunden. Der Patient PA1 erfährt von den Nutzern NA1, NB1 außerhalb des Fahrzeugs F1 eine Erstbehandlung und wurde von diesen auf eine zuvor aus dem Fahrzeug F1 entnommene Trage TR1 gelegt. Sobald die Trage TR1 mit dem auf dieser liegenden Patient PA1 in das Fahrzeug F1 eingeschoben ist, liegt der Patient PA1 in einem Aufnahmebereich AN1 der Digitalkameras A1, B1.

In der Figur 2 ist eine zweite Ausführungsvariante eines erfindungsgemäßen Telenotarztsystems TAS2 gezeigt, welches - wie die erste Ausführungsvariante - nur einen Router RA1 umfasst und welches aber zusätzlich eine medizintechnische Ausrüstung ME2 umfasst. Insofern wird zu der Figur 2 grundsätzlich auf die oben stehende Beschreibung zu der Figur 1 verwiesen, wobei vergleichbare Bauteile mit gleichen Bezugszeichen bezeichnet sind. Bei der medizinischen Ausrüstung ME2 handelt es sich um Ausrüstungsgegenstände wie z.B. Pulsmesser und Blutdruckmesser. Diese Ausrüstungsgegenstände sind drahtlos oder drahtgebunden an den Router RA1 angeschlossen, so dass die von diesen Ausrüstungsgegenständen erfassten Vitaldaten über den Router RA1 an den Server SV1 weitergeleitet werden. Die Kommunikation mit dem Router RA1 erfolgt hierbei entweder direkt durch jeden Ausrüstungsgegenstand oder über einen der Ausrüstungsgegenstände oder über eine zusätzlich Kommunikationseinheit, welche von der medizinischen Ausrüstung ME2 umfasst ist.

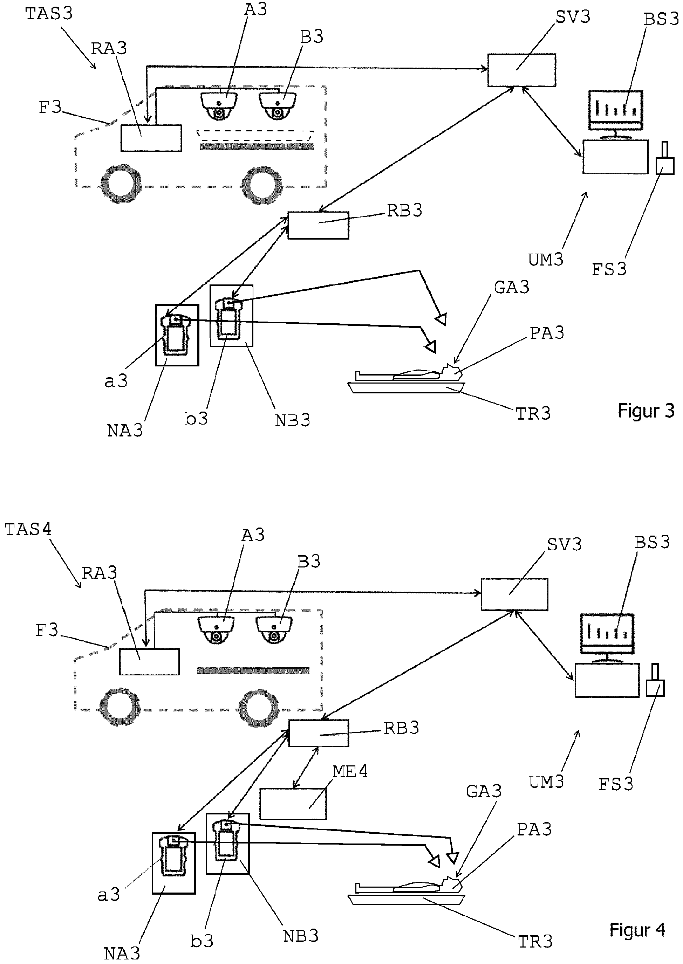

In der Figur 3 ist eine dritte Ausführungsvariante eines erfindungsgemäßen Telenotarztsystems TAS3 gezeigt, welches einen ersten Router RA1 und einen zweiten Router RB2 umfasst. Das Telenotarztsystem TAS3 umfasst eine erste in einem Fahrzeug F3 angeordnete Digitalkamera A3 und eine zweite in dem Fahrzeug F3 angeordnete Kamera B3. Weiterhin umfasst das Telenotarztsystem TAS3 zwei tragbare Digitalkameras a3, b3, wobei ein Nutzer NA3 die erste tragbare Digitalkamera a3 trägt und wobei ein Nutzer NB3 die zweite Digitalkamera b3 trägt. Das Telenotarztsystem TAS3 umfasst auch ein Überwachungsmodul UM3, welches einen Bildschirm BS3 und ein Fernsteuereinheit FS3 umfasst. Weiterhin umfasst das Telenotarztsystem TAS3 den erwähnten ersten Router RA3, mit welchem die im Fahrzeug F1 angeordneten Digitalkameras A3, B3 verbunden sind, sowie den erwähnten zweiten Router RB3, mit welchem die tragbaren Digitalkameras a3, b3 verbunden sind. Schließlich umfasst das Telenotarztsystem TAS3 einen Server SV3, mit welchem der erste Router RA3, der zweite Router RB3 und das Überwachungsmodul UM3 verbunden sind. Beide in dem Fahrzeug F3 angeordnete Digitalkameras A3, B3 umfassen als Steuermechanismen einen Zoom-Mechanismus und einen Schwenk- /Drehmechanismus und eine elektronische Bild-Ausschnittsvorrichtung, wobei die Steuermechanismen der in dem Fahrzeug F3 angeordneten Digitalkameras A3, B3 mittels der Fernsteuereinheit FS3 des Überwachungsmoduls UM3 ferngesteuert sind. Der Sever SV3 generiert auf der Basis der von den tragbaren Digitalkameras a3, b3 aufgenommenen Digitalbilder eine Kollektionen wesentlicher Merkmale des aufgenommenen Patienten PA3, wobei die Kollektion wesentlicher Merkmale insbesondere jeweils aus einem Gesicht GA3 des Patienten PA3 erzeugt ist. Ein Algorithmus vergleicht die Kollektion der wesentlichen Merkmale des Patienten PA3 mit den laufend in dem Fahrzeug F3 von den beiden Digitalkameras A3, B3 aufgenommenen Digitalbildern derart, dass das Telenotarztsystem TAS3 eine Übertragung der in dem Fahrzeug F3 aufgenommenen Digitalbilder an den Bildschirm BS3 des Überwachungsmoduls UM3 startet, wenn ein Vergleich ergibt, dass der von den tragbaren Digitalkameras a3, b3 erfasste Patienten PA3 von einer der in dem Fahrzeug F3 angeordneten Digitalkameras A3, B3 erfasst ist.

Hierbei ist der Algorithmus gemäß einer ersten Ausführungsvariante in den ersten Router RA3 implementiert.

Hierbei ist der Algorithmus gemäß einer zweiten Ausführungsvariante in den zweiten Router RB3 implementiert. Sowohl der erste Router RA3 als auch der zweite Router RB3 ist mit dem Server SV1 drahtlos über ein Mobilfunknetzwerk oder über Satellitenfunk verbunden. Gemäß einer weiteren Ausführungsvariante ist der Algorithmus in den Server SV3 implementiert. Der zweite Router RB3 ist als mobiler Router ausgebildet, welcher bei einer Erstversorgung des Patienten PA3 außerhalb des Fahrzeugs F3 aufgestellt ist. Hierzu verfügt der zweite Router RB3 über eine eigene Energieversorgung. Der Patient PA3 erfährt von den Nutzern NA3, NB3 außerhalb des Fahrzeugs F3 eine Erstbehandlung und wurde von diesen auf eine zuvor aus dem Fahrzeug F3 entnommene Trage TR3 gelegt. Sobald die Trage TR3 mit dem auf dieser liegenden Patient PA3 in das Fahrzeug F3 eingeschoben ist, liegt der Patient PA3 in einem Aufnahmebereich AN3 der Digitalkameras A3, B3.

In der Figur 4 ist eine vierte Ausführungsvariante eines erfindungsgemäßen Telenotarztsystems TAS4 gezeigt, welches - wie die dritte Ausführungsvariante - einen ersten Router RA3 und einen zweiten Router RB3 umfasst und welches zusätzlich eine medizintechnische Ausrüstung ME4 umfasst. Insofern wird hier auf die oben stehende Beschreibung zu der Figur 3 verwiesen, wobei vergleichbare Bauteile mit gleichen Bezugszeichen bezeichnet sind. Bei der medizinischen Ausrüstung ME4 handelt es sich um Ausrüstungsgegenstände wie z.B. Pulsmesser und Blutdruckmesser. Diese Ausrüstungsgegenstände sind drahtlos oder drahtgebunden an den zweiten Router RB3 angeschlossen, so dass die von diesen erfassten Vitaldaten über den zweiten Router RB3 an den Server SV3 weitergeleitet werden. Die Kommunikation mit dem zweiten Router RB3 erfolgt hierbei entweder direkt durch jeden Ausrüstungsgegenstand oder über einen der Ausrüstungsgegenstände oder über eine zusätzlich Kommunikationseinheit, welche von der medizinischen Ausrüstung ME4 umfasst ist. Es ist insbesondere auch vorgesehen, weitere medizinische Vorrichtungen wie zum Beispiel ein Bluetooth Stethoskop mit der Digitalkamera A3 und/oder mit der Digitalkamera B3 zu verbinden und von der jeweiligen medizinischen Vorrichtung erfasste Vitaldaten in einem Audiokanal von der Digitalkamera A3 und/oder der Digitalkamera B3 zu einem der Router RA3 oder RB3 zu übertragen.

Allgemein kann zu den vier Ausführungsbeispielen festgehalten werden: Bei einem Telenotarztsystem für Fahrzeuge, welches mindesten eine fernsteuerbare, im Fahrzeug angeordnete Digitalkamera umfasst, welche auf den zu transportierenden Patienten fokussiert ist, und welches wenigstens eine tragbare Digitalkamera umfasst, die von wenigstens einem Nutzer, nämlich einem Rettungssanitäter, an einem Einsatzort und beim Transport des Patienten zum Fahrzeug getragen wird, wird für einen Bildschirm eines Überwachungsmoduls eine Umschaltung von Digitalbildern, welche von der tragbaren Digitalkamera geliefert werden, auf Digitalbilder, welche von der im Fahrzeug angeordneten Digitalkamera geliefert werden, vorgenommen. Hierbei ist das Fahrzeug als Ambulanzfahrzeug ausgebildet. Wenn der Patient in das Fahrzeug verbracht ist, erfolgt die Umschaltung auf wenigstens eine der in dem Fahrzeug angeordneten Digitalkameras automatisch, so dass die Nutzer, welche in der Regel Rettungssanitäter sind, nicht mit zusätzlichen Aufgaben belastet sind.

Entsprechend wird mittels der wenigstens einer tragbaren Digitalkamera, die von dem Nutzer, welche dieser trägt, regelmäßig auf den Patienten gerichtet ist, eine Kollektion wesentlicher Merkmale des Gesichtes des Patienten erfasst, wobei dies entweder in der tragbaren Digitalkamera selbst oder dem einen Router oder einem der Router erfolgt oder in dem Server erfolgt, zu dem eine ständige Verbindung besteht.

In dieser Phase, in welcher der Patient außerhalb des Fahrzeugs von den Rettungssanitätern behandelt wird, werden entsprechend wesentliche Merkmale des Gesichts des Patienten erkannt, ständig verifiziert und optional mit der ggf. vorhandenen weiteren tragbare Digitalkamera des weiteren Rettungssanitäters abgeglichen. Diese Kollektion von Merkmalen wird wiederholt an die in dem Fahrzeug verbaute Digitalkamera übergeben und von dem Telenotarztsystem mit dem Digitalbild aus dem Fahrzeug verglichen. Sobald der Patient im Fahrzeug wiedererkannt wird, erfolgt nach einer entsprechenden Signalisierung die automatische Umschaltung auf die im Fahrzeug verbaute Digitalkamera und die Digitalbilder dieser im Fahrzeug verbauten Digitalkamera werden an das Überwachungsmodul übermittelt.

Die Gesichtserkennung wird wahlweise von der in dem Fahrzeug verbauten Digitalkamera oder von dem Router, an welchen die in dem Fahrzeug verbaute Digitalkamera angeschlossen ist, oder von dem Server ausgeführt.

Es ist vorgesehen die in dem Fahrzeug verbaute Digitalkamera zu aktivieren, wenn die Trage aus dem Fahrzeug entnommen wird oder dadurch zu aktivieren, dass am Einsatzort durch eine der tragbaren Digitalkameras eine Übertragung initiiert wird.

### Bezugszeichenliste:

- TAS1: erstes Telenotarztsystem
- RA1: Router
- F1: Fahrzeug
- A1: erste Digitalkamera
- B1: Digitalkamera
- a1: erste tragbare Digitalkamera
- b1: zweite tragbare Digitalkamera
- NA1: erster Nutzer
- NB2: zweiter Nutzer
- UM1: Überwachungsmodul
- BS1: Bildschirm
- FS1: Fernsteuereinheit
- SV1: Server
- PA1: Patient
- GA1: Gesicht des Patienten
- TR1: Trage

- TAS2: zweites Telenotarztsystem
- ME2: medizinische Ausrüstung

- TAS3: drittes Telenotarztsystem
- RA3: erster Router
- RB3: zweiter Router
- F3: Fahrzeug
- A3: erste Digitalkamera
- B3: Digitalkamera
- a3: erste tragbare Digitalkamera
- b3: zweite tragbare Digitalkamera
- NA3: erster Nutzer
- NB3: zweiter Nutzer
- UM3: Überwachungsmodul
- BS3: Bildschirm
- FS3: Fernsteuereinheit
- SV3 BY: Server
- PA3: Patient
- GA3: Gesicht des Patienten
- TR3: Trage

- TAS4: viertes Telenotarztsystem
- ME4: medizinische Ausrüstung

## Patentansprüche

1. Telenotarztsystem (TAS1; TAS2; TAS3; TAS4) umfassend
- wenigstens eine in einem Fahrzeug (F1; F3) angeordnete Digitalkamera (A1, B1; A3; B3),
- wenigstens eine tragbare Digitalkamera (a1, b1; a3, b3), die von einem Nutzer (NA1, NA2; NA3, NB3) getragen ist,
- ein Überwachungsmodul (UM1; UM3), welches einen Bildschirm (BS1; BS3) und ein Fernsteuereinheit (FS1; FS3) umfasst,
- wenigstens einen Router (RA1; RA3, RB3), mit welchem die Digitalkameras (A1, B1, a1, b1; A3, B3, a3, b3) verbunden sind,
- einen Server (SV1; SV3), mit welchem der wenigstens eine Router (RA1; RA3, RB3) und das Überwachungsmodul (UM1; UM2) verbunden sind,
- wobei die wenigstens eine in dem Fahrzeug (F1; F3) angeordnete Digitalkamera (A1, B1; A3, B3) als Steuermechanismen einen Zoom-Mechanismus und/oder einen Schwenk- /Drehmechanismus umfasst,
- wobei die Steuermechanismen der wenigstens einen in dem Fahrzeug (F1; F3) angeordneten Digitalkamera (A1, B1; A3, B3) mittels der Fernsteuereinheit (FS1; FS3) des Überwachungsmoduls (UM1; UM3) ferngesteuert sind,
**dadurch gekennzeichnet, dass**
- die wenigstens eine tragbare Digitalkamera (a1, b1; a3, b3) oder wenigstens einer der Router (RA1; RA3, RB3) oder der Server (SV1; SV3) auf der Basis der von der wenigstens einen tragbaren Digitalkamera (a1, b1; a3, b3) aufgenommenen Digitalbilder eine Kollektion wesentlicher Merkmale wenigstens eines Patienten (PA1; PA3) generiert, wobei die Kollektion wesentlicher Merkmale insbesondere aus einem Gesicht (GE1; GE3) des Patienten (PA1; PA3) generiert ist,
- wobei ein Algorithmus die Kollektion der wesentlichen Merkmale des Patienten (PA1; PA3) mit den laufend in dem Fahrzeug (F1; F3) von der wenigstens einen Digitalkamera (A1, B1; A3, B3) aufgenommenen Digitalbildern derart vergleicht, dass das Telenotarztsystem (TAS1; TAS2; TAS3; TAS4) eine Übertragung der in dem Fahrzeug (F1; F3) aufgenommenen Digitalbilder an den Bildschirm (BS1; BS3) des Überwachungsmoduls (UM1; UM3) startet, sobald ein Vergleich ergibt, dass einer der von der tragbaren Digitalkamera (a1, b1; a3, b3) erfassten Patienten (PA1; PA3) von der in dem Fahrzeug (F1; F3) angeordneten Digitalkamera (A1, B1; A3, B3) erfasst ist,
- wobei der Algorithmus in wenigstens eine der Digitalkameras (A1, B1; A3, B3) des Fahrzeugs (F1; F3) oder in den wenigstens einen der Router (RA1; RA3, RB3) oder in den Server (SV1; SV3) implementiert ist.

2. Telenotarztsystem (TAS2; TAS4) nach Anspruch 1, **dadurch gekennzeichnet, dass** das Telenotarztsystem (TAS2; TAS4) eine medizintechnische Ausrüstung (ME2; ME4) umfasst, wobei diese außerhalb des Fahrzeugs (F1; F3) angeordnet ist, wobei diese zur Erfassung von Vitaldaten des Patienten (PA1; PA3) geeignet ist und wobei diese über den wenigstens einen Router (RA1; RA3, RB3) mit dem Server (SV1; SV3) derart verbunden ist, dass die erfassten Vitaldaten an den Server (SV1; SV3) übertragen werden.

3. Telenotarztsystem (TAS1; TAS2; TAS3; TAS4) nach wenigstens einem der vorhergehenden Ansprüche, **dadurch gekennzeichnet, dass** der wenigstens eine Router (RA1; RA3, RB3) mit dem Server (SV1; SV3) drahtlos kommuniziert.

4. Telenotarztsystem (TAS3; TAS4) nach wenigstens einem der vorhergehenden Ansprüche, **dadurch gekennzeichnet, dass** das Telenotarztsystem (TAS3; TAS4) zwei Router (RA3, RB3) umfasst, wobei die in dem Fahrzeug (F1; F3) angeordnete wenigstens eine Digitalkamera (A3, B3) mit dem ersten Router (RA3) verbunden ist, wobei der erste Router (RA1; RA3) in dem Fahrzeug (F1; F3) angeordnet ist, wobei wenigstens ein der von den Nutzern (NA3, NB3) getragenen Digitalkameras (a3, b3) mit dem zweiten Router (RB3) verbunden ist, wobei der zweite Router (RB3) als portabler Router ausgebildet ist und im Einsatz außerhalb des Fahrzeugs (F3) angeordnet ist.

5. Verfahren zum Betrieb eines Telenotarztsystems (TAS1; TAS2; TAS3; TAS4), welches nach wenigstens einem der Ansprüche 1 bis 4 ausgebildet ist, umfassend die Schritte:
- Filmen eines Patienten (PA1; PA3) mit einer von einem Nutzer (NA1, NB1; NA3, NB3) getragenen tragbaren Digitalkamera (a1, b1; a3, b3);
und **gekennzeichnet durch**:
- Generieren einer Kollektion wesentlicher Merkmale eines Patienten (PA1; PA3) auf der Basis der durch das Filmen erfassten Digitalbilder, wobei die Kollektion wesentlicher Merkmale insbesondere aus einem Gesicht (GE1; GE3) des Patienten (PA1; PA3) generiert wird und wobei die Generierung der Kollektion durch die tragbare Digitalkamera (a1, b1; a3, b3) oder durch wenigstens einen der Router (RA1; RA3, RB3) oder durch den Server (SV1; SV3) erfolgt;
- Vergleichen der Kollektion der wesentlichen Merkmale des Patienten (PA1; PA3) mit den laufend in dem Fahrzeug (F1; F3) von einer dortigen Digitalkamera (A1, B1; A3, B3) aufgenommenen Digitalbildern mittels eines Algorithmus, wobei der Algorithmus in die Digitalkameras (A1, B1; A3, B3) des Fahrzeugs (F1; F3) oder in den Router (RA1; RA3, RB3) oder in den Server (SV1; SV3) implementiert ist;
- Starten einer Übertragung der in dem Fahrzeug (F1; F3) aufgenommenen Digitalbilder an einen Bildschirm (BS1; BS3) eines Überwachungsmoduls (UM1; UM3), sobald das Vergleichen zu dem Ergebnis führt, dass der von der tragbaren Digitalkamera (a1, b1; a3, b3) bereits erfasste Patient (PA1) von der in dem Fahrzeug (F1; F3) angeordneten Digitalkamera (A1, B1; A3, B3) erfasst wird.

6. Verfahren zum Betrieb eines Telenotarztsystems (TAS1; TAS2; TAS3; TAS4) nach Anspruch 5 umfassend die zusätzlichen Schritte:
- Erfassen von Vitaldaten des Patienten (PA1; PA3) während der Patient von dem Nutzer (NA1, NB1; NA3; NB3) mit der tragbaren Digitalkamera (a1, b1; a3, b3) aufgenommen wird;
- Übermitteln der Vitaldaten des Patienten (PA1; PA3) zu dem Router (RA1; RB3) und von dem Router (RA1; RB3) zu dem Server (SV1; SV3).

7. Verfahren zum Betrieb eines Telenotarztsystems (TAS1; TAS2; TAS3; TAS4) nach Anspruch 6 umfassend den zusätzlichen Schritt:
- Einblenden der an den Server (SV1; SV3) übermittelten Vitaldaten des Patienten (PA1; PA3) in ein auf einem Bildschirm (BS1; BS3) eines Überwachungsmoduls (UM1; UM3) angezeigtes Bild des Patienten (PA).

8. Verfahren zum Betrieb eines Telenotarztsystems (TAS1; TAS2; TAS3; TAS4) nach wenigstens einem der Ansprüche 5, 6 oder 7 umfassend den zusätzlichen Schritt:
- Aktivieren wenigstens einer der in dem Fahrzeug (F1; F3) angeordneten Digitalkameras (A1, B1; A3, B3) durch Entnahme einer Trage (TR1; TR3) aus dem Fahrzeug (F1; F3).

9. Verfahren zum Betrieb eines Telenotarztsystems (TAS1; TAS2; TAS3; TAS4) nach wenigstens einem der Ansprüche 5, 6 oder 7 umfassend den zusätzlichen Schritt:
- Aktivierung der wenigstens einen in dem Fahrzeug (F1; F3) angeordneten Digitalkamera (A1, B1; A3, B3) durch Aktivierung einer Live-Video Funktion an einer der tragbaren Digitalkameras (a1, b1; a3, b3).

10. Verfahren zum Betrieb eines Telenotarztsystems (TAS1; TAS2; TAS3; TAS4)nach wenigstens einem der vorgenannten Verfahrensansprüche umfassend die Schritte:
- Erfassen von Vitaldaten mit einer medizinischen Vorrichtung wie zum Beispiel insbesondere einem Stethoskop;
- Übertragung der Vitaldaten von der medizinischen Vorrichtung an eine in einem Fahrzeug (F1; F3) angeordnete Digitalkamera (A1, B1; A3, B3) per Funk, insbesondere per Bluetooth;
- Weiterleitung der Vitaldaten über einen Audiokanal der Digitalkamera (A1, B1; A3, B3) an einen Router (RA1; RA3, RB3) .

## Claims

1. Remote emergency doctor system (TAS1; TAS2; TAS3; TAS4) comprising
- at least one digital camera (A1, B1; A3; B3) installed in a vehicle (F1; F3),
- at least one portable digital camera (a1, b1; a3, b3) carried by a user (NA1, NA2; NA3, NB3),
- a monitoring module (UM1; UM3) comprising a display screen (BS1; BS3) and a remote control unit (FS1; FS3),
- at least one router (RA1; RA3, RB3) to which the digital cameras (A1, B1, a1, b1; A3, B3, a3, b3) are connected,
- a server (SV1; SV3) to which the at least one router (RA1; RA3, RB3) and the monitoring module (UM1; UM2) are connected,
- wherein the at least one digital camera (A1, B1; A3, B3) installed in the vehicle (F1; F3) comprises a zoom mechanism and/or a pivot/rotation mechanism as control mechanisms,
- wherein the control mechanisms of the at least one digital camera (A1, B1; A3, B3) installed in the vehicle (F1; F3) are remotely controlled by means of the remote control unit (FS1; FS3) of the monitoring module (UM1; UM3),
**characterized in that**
- the at least one portable digital camera (a1, b1; a3, b3) or at least one of the routers (RA1; RA3, RB3) or the server (SV1; SV3) generates a collection of essential features of at least one patient (PA1; PA3) on the basis of the digital images recorded by the at least one portable digital camera (a1, b1; a3, b3), the collection of essential features being generated, in particular, from a face (GE1; GE3) of the patient (PA1; PA3),
- wherein an algorithm compares the collection of the essential features of the patient (PA1; PA3) with the digital images continuously recorded in the vehicle (F1; F3) by the at least one digital camera (A1, B1; A3, B3), in such a way that the remote emergency doctor system (TAS1; TAS2; TAS3; TAS4) starts to transfer the digital images recorded in the vehicle (F1; F3) to the display screen (BS1; BS3) of the monitoring module (UM1; UM3) as soon as a comparison shows that one of the patients (PA1; PA3) detected by the portable digital camera (a1, b1; a3, b3) is detected by the digital camera (A1, B1; A3, B3) installed in the vehicle (F1; F3),
- wherein the algorithm is implemented in at least one of the digital cameras (A1, B1; A3, B3) of the vehicle (F1; F3) or in the at least one of the routers (RA1; RA3, RB3) or in the server (SV1; SV3).

2. Remote emergency doctor system (TAS2; TAS4) according to Claim 1, **characterized in that** the remote emergency doctor system (TAS2; TAS4) comprises a medical device (ME2; ME4), said device being arranged outside the vehicle (F1; F3), wherein said device is suitable for acquiring vital data of the patient (PA1; PA3) and is connected to the server (SV1; SV3) via the at least one router (RA1; RA3, RB3) in such a way that the vital data acquired is transmitted to the server (SV1; SV3).

3. Remote emergency doctor system (TAS1; TAS2; TAS3; TAS4) according to at least one of the previous claims, **characterized in that** the at least one router (RA1; RA3, RB3) communicates wirelessly with the server (SV1; SV3).

4. Remote emergency doctor system (TAS3; TAS4) according to at least one of the previous claims, **characterized in that** the remote emergency doctor system (TAS3; TAS4) comprises two routers (RA3, RB3), wherein the at least one digital camera (A3, B3) installed in the vehicle (F1; F3) is connected to the first router (RA3), wherein the first router (RA1; RA3) is arranged in the vehicle (F1; F3), wherein at least one of the digital cameras (a3, b3) carried by the users (NA3, NB3) is connected to the second router (RB3), the second router (RB3) being designed as a portable router and arranged outside the vehicle (F3) when in use.

5. Method for operating a remote emergency doctor system (TAS1; TAS2; TAS3; TAS4), which is designed according to at least one of Claims 1 to 4, comprising the steps:
- filming a patient (PA1; PA3) with a portable digital camera (a1, b1; a3, b3) worn by a user (NA1, NB1; NA3, NB3) ;
and **characterized by**:
- generating a collection of essential features of a patient (PA1; PA3) on the basis of the digital images acquired by the filming, the collection of essential features being generated, in particular, from a face (GE1; GE3) of the patient (PA1; PA3) and the collection being generated by the portable digital camera (a1, b1; a3, b3) or by at least one of the routers (RA1; RA3, RB3) or by the server (SV1; SV3);
- comparing the collection of the essential features of the patient (PA1; PA3) with the digital images continuously recorded in the vehicle (F1; F3) by a local digital camera (A1, B1; A3, B3) by means of an algorithm, the algorithm being implemented in the digital cameras (A1, B1; A3, B3) of the vehicle (F1; F3) or in the router (RA1; RA3, RB3) or in the server (SV1; SV3);
- starting a transfer of the digital images recorded in the vehicle (F1; F3) to a display screen (BS1; BS3) of a monitoring module (UM1; UM3) as soon as the result of the comparison shows that the patient (PA1) already detected by the portable digital camera (a1, b1; a3, b3) is detected by the digital camera (A1, B1; A3, B3) installed in the vehicle (F1; F3).

6. Method for operating a remote emergency doctor system (TAS1; TAS2; TAS3; TAS4) according to Claim 5, comprising the additional steps:
- acquiring vital data of the patient (PA1; PA3) while the patient is being recorded by the user (NA1, NB1; NA3; NB3) using the portable digital camera (a1, b1; a3, b3);
- transferring the vital data of the patient (PA1; PA3) to the router (RA1; RB3) and from the router (RA1; RB3) to the server (SV1; SV3).

7. Method for operating a remote emergency doctor system (TAS1; TAS2; TAS3; TAS4) according to Claim 6, comprising the additional step:
- overlaying the vital data of the patient (PA1; PA3) transmitted to the server (SV1; SV3) onto an image of the patient (PA) displayed on a display screen (BS1; BS3) of a monitoring module (UM1; UM3).

8. Method for operating a remote emergency doctor system (TAS1; TAS2; TAS3; TAS4) according to at least one of Claims 5, 6 or 7, comprising the additional step:
- activating at least one of the digital cameras (A1, B1; A3, B3) installed in the vehicle (F1; F3) by removing a stretcher (TR1; TR3) from the vehicle (F1; F3).

9. Method for operating a remote emergency doctor system (TAS1; TAS2; TAS3; TAS4) according to at least one of Claims 5, 6 or 7, comprising the additional step:
- activating the at least one digital camera (A1, B1; A3, B3) installed in the vehicle (F1; F3) by activating a live video function on one of the portable digital cameras (a1, b1; a3, b3).

10. Method for operating a remote emergency doctor system (TAS1; TAS2; TAS3; TAS4) according to at least one of the aforementioned method claims, comprising the steps:
- acquiring vital data using a medical device, such as a stethoscope in particular,
- transmitting the vital data from the medical device to a digital camera (A1, B1; A3, B3) installed in a vehicle (F1; F3) by radio, in particular via Bluetooth;
- forwarding the vital data via an audio channel of the digital camera (A1, B1; A3, B3) to a router (RA1; RA3, RB3).

## Revendications

1. Système de télémédecine d'urgence (TAS1 ; TAS2 ; TAS3 ; TAS4) comprenant
- au moins une caméra numérique (A1, B1 ; A3 ; B3) disposée dans un véhicule (F1 ; F3),
- au moins une caméra numérique portable (a1, b1 ; a3, b3) qui est portée par un utilisateur (NA1, NA2 ; NA3, NB3),
- un module de surveillance (UM1 ; UM3) qui comprend un écran (BS1 ; BS3) et une unité de télécommande (FS1 ; FS3),
- au moins un routeur (RA1 ; RA3, RB3) auquel les caméras numériques (A1, B1, a1, b1 ; A3, B3, a3, b3) sont reliées,
- un serveur (SV1 ; SV3) auquel l'au moins un routeur (RA1 ; RA3, RB3) et le module de surveillance (UM1 ; UM2) sont reliés,
- l'au moins une caméra numérique (A1, B1 ; A3, B3) disposée dans le véhicule (F1; F3) comprenant un mécanisme de zoom et/ou un mécanisme de pivotement/rotation comme mécanismes de commande,
- les mécanismes de commande de l'au moins une caméra numérique (A1, B1 ; A3, B3) disposée dans le véhicule (F1; F3) étant télécommandés au moyen de l'unité de télécommande (FS1 ; FS3) du module de surveillance (UM1 ; UM3),
**caractérisé en ce que**
- l'au moins une caméra numérique portable (a1, b1 ; a3, b3) ou l'un au moins des routeurs (RA1 ; RA3, RB3) ou le serveur (SV1 ; SV3) génèrent sur la base des images numériques capturées par l'au moins une caméra numérique portable (a1, b1 ; a3, b3) une collection de caractéristiques essentielles d'au moins un patient (PA1 ; PA3), la collection de caractéristiques essentielles étant générée notamment à partir du visage (GE1 ; GE3) du patient (PA1 ; PA3),
- un algorithme comparant la collecte des caractéristiques essentielles du patient (PA1 ; PA3) aux images numériques capturées en continu dans le véhicule (F1 ; F3) par l'au moins une caméra numérique (A1, B1 ; A3, B3) de manière à ce que le système de télémédecine (TAS1 ; TAS2 ; TAS3 ; TAS4) démarre une transmission des images numériques, capturées dans le véhicule (F1 ; F3), à l'écran (BS1 ; BS3) du module de surveillance (UM1 ; UM3) dès qu'une comparaison montre qu'un des patients (PA1 ; PA3) capturés par la caméra numérique portable (a1, b1 ; a3, b3) est capturé par la caméra numérique (A1, B1 ; A3, B3) disposée dans le véhicule (F1 ; F3),
- l'algorithme étant implémenté dans au moins une des caméras numériques (A1, B1 ; A3, B3) du véhicule (F1 ; F3) ou dans au moins un des routeurs (RA1 ; RA3, RB3) ou dans le serveur (SV1 ; SV3).

2. Système de télémédecine (TAS2 ; TAS4) selon la revendication 1, **caractérisé en ce que** le système de télémédecine (TAS2 ; TAS4) comprend un équipement de technique médicale (ME2 ; ME4), ledit équipement étant disposé à l'extérieur du véhicule (F1; F3), ledit équipement étant approprié à l'acquisition de données vitales du patient (PA1 ; PA3) et ledit équipement étant relié au serveur (SV1 ; SV3) par le biais d'au moins un routeur (RA1 ; RA3, RB3) de manière à ce que les données vitales acquises soient transmises au serveur (SV1 ; SV3).

3. Système de télémédecine (TAS1 ; TAS2 ; TAS3 ; TAS4) selon l'une au moins des revendications précédentes, **caractérisé en ce que** l'au moins un routeur (RA1 ; RA3, RB3) communique sans fil avec le serveur (SV1 ; SV3).

4. Système de télémédecine (TAS3 ; TAS4) selon l'une au moins des revendications précédentes, **caractérisé en ce que** le système de télémédecine (TAS3 ; TAS4) comprend deux routeurs (RA3, RB3), l'au moins une caméra numérique (A3, B3) disposée dans le véhicule (F1 ; F3) étant reliée au premier routeur (RA3), le premier routeur (RA1 ; RA3) étant disposé dans le véhicule (F1 ; F3), au moins une des caméras numériques (a3, b3) portées par les utilisateurs (NA3, NB3) étant reliée au deuxième routeur (RB3), le deuxième routeur (RB3) étant conçu comme un routeur portable et étant disposé à l'extérieur du véhicule (F3) lorsqu'il est utilisé.

5. Procédé de fonctionnement d'un système de télémédecine (TAS1 ; TAS2 ; TAS3 ; TAS4), conçu selon l'une au moins des revendications 1 à 4, ledit procédé comprenant les étapes suivantes :
- filmer un patient (PA1 ; PA3) avec une caméra numérique portable (a1, b1; a3, b3) portée par un utilisateur (NA1, NB1 ; NA3, NB3) ;
et **caractérisé par** les étapes suivantes :
- générer une collection de caractéristiques essentielles d'un patient (PA1 ; PA3) sur la base des images numériques capturées par l'action de filmer, la collection des caractéristiques essentielles étant générée notamment à partir du visage (GE1 ; GE3) du patient (PA1 ; PA3) et la génération de la collection étant effectuée par la caméra numérique portable (a1, b1 ; a3, b3) ou par au moins un des routeurs (RA1 ; RA3, RB3) ou par le serveur (SV1 ; SV3) ;
- comparer, à l'aide d'un algorithme, la collection des caractéristiques essentielles du patient (PA1 ; PA3) aux images numériques capturées en continu dans le véhicule (F1 ; F3) par une caméra numérique (A1, B1 ; A3, B3) située dans celui-ci, l'algorithme étant implémenté dans les caméras numériques (A1, B1 ; A3, B3) du véhicule (F1 ; F3) ou dans le routeur (RA1 ; RA3, RB3) ou dans le serveur (SV1 ; SV3) ;
- démarrer une transmission des images numériques capturées dans le véhicule (F1 ; F3) à un écran (BS1 ; BS3) d'un module de surveillance (UM1 ; UM3) dès que la comparaison conduit au résultat que le patient (PA1) déjà capturé par la caméra numérique portable (a1, b1 ; a3, b3) est capturé par la caméra numérique (A1, B1 ; A3, B3) disposée dans le véhicule (F1 ; F3).

6. Procédé de fonctionnement d'un système de télémédecine (TAS1 ; TAS2 ; TAS3 ; TAS4) selon la revendication 5, ledit procédé comprenant les étapes supplémentaires suivantes :
- acquérir des données vitales du patient (PA1 ; PA3) pendant que le patient est capturé par l'utilisateur (NA1, NB1 ; NA3 ; NB3) à l'aide de la caméra numérique portable (a1, b1 ; a3, b3) ;
- transmettre des données vitales du patient (PA1 ; PA3) au routeur (RA1 ; RB3) et du routeur (RA1 ; RB3) au serveur (SV1 ; SV3).

7. Procédé de fonctionnement d'un système de télémédecine (TAS1 ; TAS2 ; TAS3 ; TAS4) selon la revendication 6, ledit procédé comprenant l'étape supplémentaire suivante :
- incorporer les données vitales du patient (PA1 ; PA3) transmises au serveur (SV1 ; SV3) dans une image du patient (PA) affichée sur un écran (BS1 ; BS3) d'un module de surveillance (UM1 ; UM3).

8. Procédé de fonctionnement d'un système de télémédecine (TAS1 ; TAS2 ; TAS3 ; TAS4) selon l'une au moins des revendications 5, 6 ou 7, ledit procédé comprenant l'étape supplémentaire suivante :
- activer au moins une des caméras numériques (A1, B1 ; A3, B3) disposées dans le véhicule (F1 ; F3) par retrait d'une civière (TR1; TR3) du véhicule (F1 ; F3) .

9. Procédé de fonctionnement d'un système de télémédecine (TAS1 ; TAS2 ; TAS3 ; TAS4) selon l'une au moins des revendications 5, 6 ou 7, ledit procédé comprenant l'étape supplémentaire suivante :
- activer au moins une des caméras numériques (A1, B1 ; A3, B3) disposées dans le véhicule (F1 ; F3) par activation d'une fonction Live Vidéo sur l'une des caméras numériques portables (a1, b1 ; a3, b3).

10. Procédé de fonctionnement d'un système de télémédecine (TAS1 ; TAS2 ; TAS3 ; TAS4) selon l'une au moins des revendications de procédé susmentionnées, ledit procédé comprenant les étapes suivantes :
- acquérir des données vitales à l'aide d'un dispositif médical tel que par exemple un stéthoscope ;
- transmettre les données vitales du dispositif médical à une caméra numérique (A1, B1 ; A3, B3) disposée dans un véhicule (F1 ; F3) par radio, notamment par Bluetooth ;
- transmettre les données vitales sur un canal audio de la caméra numérique (A1, B1 ; A3, B3) à un routeur (RA1 ; RA3, RB3).
